# DEMANDE DE BREVET EUROPEEN

(11) **EP 3 644 137 A1**
(43) Date de publication de la demande: **29.04.2020**
(21) Numéro de dépôt: 18202784.7
(22) Date de dépôt: 26.10.2018
(51) Int. Cl.: G04G 21/02, A61B 5/00, G06F 1/16, G06F 21/32

(54) **PROCÉDÉ DE SÉCURISATION D'ACCÈS À L'UTILISATION DE FONCTIONS D'UNE MONTRE**

(71) Demandeur: Tissot S.A., 2400 Le Locle (CH)
(72) Inventeur: FRANZI, Edoardo, 1400 Cheseaux-Noréaz (CH); FLEURY, Emmanuel, 2740 Moutier (CH)
(74) Mandataire: ICB SA

(57) **Abrégé**

L'invention concerne un procédé de sécurisation d'accès à l'utilisation de fonctions d'une montre comportant les étapes suivantes :
- authentification du porteur de la montre en vue d'autoriser un accès audites fonctions ;
- contrôle à période déterminée de l'évolution d'au moins une caractéristique d'attribut corporel du porteur en vue de conserver/supprimer l'autorisation d'accès audites fonctions.

## Description

### Domaine technique

La présente invention concerne un procédé de sécurisation d'accès à l'utilisation de fonctions d'une montre et un système mettant en oeuvre un tel procédé.

L'invention concerne également une montre comprenant un tel système ainsi qu'un programme d'ordinateur.

### Art antérieur

Une montre comprend un ensemble de fonctions qui peut être utilisé par le porteur. De telles fonctions peuvent concerner en plus du réglage de l'heure ou de la date, une utilisation de données confidentielles ou privées propres au porteur et utiles pour accéder à des services personnalisés. Ces données sont par exemple des clés, certificats, codes d'authentification, mots de passe et codes personnels qui permettent de réaliser une connexion sécurisée de la montre à un réseau privé d'une entreprise, une authentification auprès de serveurs sécurisés comme un serveur bancaire, ou une messagerie sécurisée pour l'envoi et la réception de courriels signés et/ou chiffrés. Dès lors, on comprend alors qu'il est important de pouvoir sécuriser l'accès à l'utilisation des fonctions d'une telle montre.

Pour ce faire, on connait dans l'état de la technique des procédés prévoyant de sécuriser l'accès à l'utilisation des fonctions d'une montre en renforçant les critères d'authentification autorisant cet accès par exemple en mettant en oeuvre des des étapes supplémentaires d'authentification relatives à de la biométrie.

Toutefois un des inconvénients majeurs de tels procédés est lié au fait qu'une fois que le porteur de la montre est authentifié il est alors possible à n'importe quel individu d'avoir accès aux fonctions de la montre notamment dans le cas où cette dernière a été volée.

On comprend qu'il existe un besoin de trouver une solution alternative, notamment qui ne présente pas les inconvénients de l'art antérieur.

### Résumé de l'invention

Un but de la présente invention est par conséquent de proposer un procédé de sécurisation d'accès à l'utilisation de fonctions d'une montre qui soit fiable et robuste.

Un tel procédé a pour avantage de s'assurer de l'identité du porteur de la montre de manière automatique, transparente et non intrusive pour le porteur.

Dans ce dessein, l'invention porte sur un procédé de sécurisation d'accès à l'utilisation de fonctions d'une montre comportant les étapes suivantes :
- authentification du porteur de la montre en vue d'autoriser un accès audites fonctions ;
- contrôle à période déterminée de l'évolution d'au moins une caractéristique d'attribut corporel du porteur en vue de conserver/supprimer l'autorisation d'accès audites fonctions.
Dans d'autres modes de réalisation :
- l'étape de contrôle comprend une sous-étape de détermination d'au moins une mesure d'un signal résultant de l'acquisition de la caractéristique d'attribut corporel du porteur ;
- l'étape de contrôle comprend une sous-étape de comparaison entre ladite au moins une mesure et un seuil de référence ;
- la sous-étape de comparaison comprend une phase de suppression de l'autorisation d'accès audites fonctions de la montre si ladite au moins une mesure est sensiblement différente ou différente du seuil de référence ;
- la sous-étape de comparaison comprend une phase de conservation de l'autorisation d'accès audites fonctions de la montre si ladite au moins une mesure est sensiblement égale ou égale au seuil de référence ;
- le procédé comprend une étape de définition d'au moins une caractéristique d'attribut corporel du porteur dont l'évolution est à contrôler ;
- le procédé comprend une étape de génération de seuils de référence relatifs aux caractéristiques d'attribut corporel du porteur authentifié, et
- la caractéristique d'attribut corporel du porteur est choisie parmi les caractéristiques physiologiques, d'expressions corporelles et/ou biométriques du porteur.

L'invention porte également sur un système de sécurisation d'accès à l'utilisation de fonctions d'une montre mettant en oeuvre un tel procédé, le système comprenant les éléments suivants reliés entre eux : une unité de traitement, au moins un élément d'acquisition de caractéristiques d'attribut corporel du porteur, une interface de saisie et des interfaces de diffusion d'une information visuelle et/ou sonore.

L'invention porte aussi sur une montre (100) comportant un tel système.

Avantageusement la montre est une montre mécanique connectée.

L'invention porte aussi un programme d'ordinateur comprenant des instructions de code de programme pour l'exécution des étapes de ce procédé lorsque ledit programme est exécuté par une unité de traitement.

### Brève description des figures

D'autres particularités et avantages ressortiront clairement de la description qui en est faite ci-après, à titre indicatif et nullement limitatif, en référence aux figures annexées, dans lesquelles :
- la figure 1 est une représentation schématique d'une montre comprenant un système de sécurisation d'accès à l'utilisation de fonctions d'une montre, selon un mode de réalisation de l'invention, et
- la figure 2 est un logigramme relatif à un procédé de sécurisation d'accès à l'utilisation de ces fonctions, selon le mode de réalisation de l'invention.

### Description détaillée de l'invention

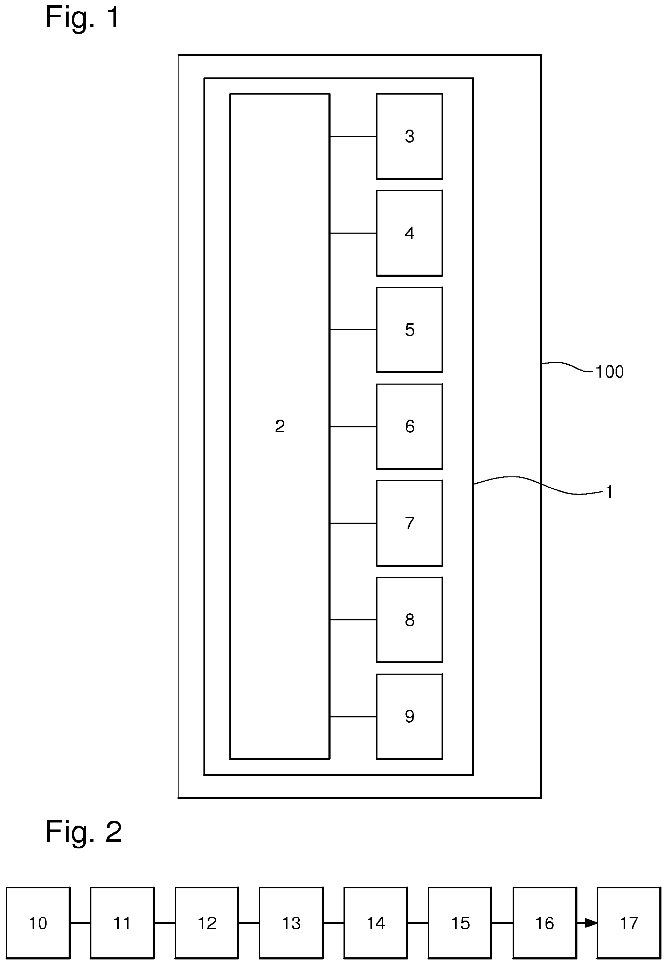

Sur la figure 1 est représenté une montre 100 comprenant un système de sécurisation d'accès à l'utilisation de fonctions de cette montre 100. Un tel système 1 est compris dans la montre 100 qui est de préférence une montre 100 mécanique connecté à affichage hybride. Ce système 1 comprend plus précisément de manière non limitative et/ou non exhaustive :
- une unité de traitement 2 comportant des ressources matérielles et logicielles en particulier au moins un processeur coopérant avec des éléments de mémoire ;
- une interface de diffusion d'une information visuelle 3 telle qu'un cadran d'affichage hybride pourvu d'une première composante d'affichage analogique et d'une deuxième composante d'affichage numérique et/ou alphanumérique ;
- une interface de diffusion d'une information sonore 4 telle qu'un haut-parleur ;
- une interface de communication ;
- une interface de saisie 9 tel qu'un clavier ou encore une interface tactile comprise par exemple dans l'interface de diffusion d'une information visuelle 3 ;
- des éléments d'acquisition 8 de caractéristiques d'attribut corporel du porteur comprenant :
   - au moins un capteur biométrique 5 ;
   - au moins un capteur d'expression corporelle 6, et
   - au moins un capteur physiologique 7.

Dans ce système 1, l'unité de traitement 2 est reliée entre autres aux interfaces de diffusion d'une information visuelle et sonore et aux éléments d'acquisition 8 de caractéristiques d'attribut corporel du porteur.

Le capteur biométrique est apte à participer à des mesures de caractéristiques physiques ou biologiques propres au porteur. Concernant le capteur d'expression corporelle 6, il est apte à acquérir de manière non limitative et non exhaustive des mouvements, gestes, démarches du porteur. Pour ce faire, ces capteurs d'expression corporelle 6 peuvent comprendre un ou plusieurs capteurs inertiels de type accéléromètre, gyroscope ou gyromètre multiaxes miniature tels que des capteurs multiaxes fabriqués en technologie MEMS, capables de détecter des vitesses angulaires et des accélérations linéaires selon plusieurs axes associant accéléromètres et/ou gyroscopes. S'agissant du capteur physiologique 7, il est apte à mesurer les paramètres relatifs au fonctionnement d'un organisme du porteur tels que, par exemple, le pouls, la saturation du sang en oxygène, l'impédance de la peau, la tension artérielle, le rythme respiratoire, l'arythmie respiratoire, la température cutanée, le taux de sudation, la saturation du sang en oxygène ou le débit sanguin.

De manière générale, chaque capteur est apte à convertir en un signal une caractéristique d'attribut corporel du porteur qu'il a acquise, lequel signal est ensuite transmis à l'unité de traitement 2 qui détermine au moins une mesure à partir d'un traitement de ce signal.

Un tel système 1 de la montre 100 est apte à mettre en oeuvre un procédé de sécurisation d'accès à l'utilisation des fonctions de la montre 100, représenté sur la figure 2. Un tel procédé vise à s'assurer de manière non intrusive et automatique de l'identité d'un porteur authentifié afin qu'il puisse utiliser les fonctions de la montre 100 tout le temps qu'il la porte sans avoir s'authentifier de nouveau. Les fonctions de la montre 100 peuvent concerner le réglage de l'heure, de la date ou encore l'utilisation d'un chronomètre, d'un système de navigation, un podomètre, d'un traceur d'activité, d'une messagerie, un accès à des données confidentielles ou privées propres au porteur et utiles pour accéder à des services personnalisés, etc...

Ce procédé comprend une étape d'authentification 10 du porteur de la montre 100 en vue d'autoriser un accès audites fonctions de cette montre 100. Cette étape 10 permet au porteur d'apporter la preuve de son identité. En effet, le porteur peut interagir avec l'interface de saisie 9 pour une authentification à partir d'un authentifiant ou d'un code secret.

Ce procédé comprend ensuite une étape de définition 12 d'au moins une caractéristique d'attribut corporel du porteur dont l'évolution est à contrôler. Cette caractéristique d'attribut corporel du porteur est choisie parmi les caractéristiques physiologiques, d'expressions corporelles et/ou biométriques du porteur. Une telle étape 12 peut être réalisée automatiquement par l'unité de traitement 2 qui définit alors un nombre aléatoire de caractéristiques d'attribut corporel du porteur dont l'évolution doit être surveillée. De manière alternative, ces caractéristiques d'attribut corporel dont l'évolution est à contrôler peuvent être sélectionnées par le porteur par l'intermédiaire de l'interface de saisie 9 et des interfaces de diffusion 3, 4. A titre d'exemple trois caractéristiques d'attribut corporel dont l'évolution est à contrôler peuvent être sélectionnées : une température, un réflexe psychogalvanique, un geste. Comme on le verra par la suite dès lors qu'une de ces caractéristiques présente une variation significative alors l'autorisation d'accès au fonction de la montre 100 est supprimée du fait que le porteur et propriétaire de la montre 100 n'est plus en sa possession. Dans ce contexte pour utiliser de nouveau les fonctions de la montre 100, il est nécessaire de s'authentifier.

Par la suite, le procédé comprend une étape de contrôle 13 à période déterminée de l'évolution d'au moins une caractéristique d'attribut corporel du porteur en vue de conserver/supprimer l'autorisation d'accès audites fonctions. Lors de cette étape, la période déterminée est une période de contrôle régulière ou irrégulière de l'évolution de la caractéristique d'attribut corporel. Cette période peut être configurée automatiquement par l'unité de traitement 2 ou définie par le porteur. A titre d'exemple, la caractéristique d'attribut corporel peut être contrôlée sur une période déterminée de quelques secondes ou quelques minutes. Une telle étape de contrôle 13 vérifie donc durant cette période l'évolution de cette caractéristique d'attribut corporel. Cette évolution de la caractéristique peut être stable c'est-à-dire qu'elle ne présente aucune variation, ou au contraire présenter une variation. Une telle évolution est déterminée comme on le verra par la suite relativement à un seuil de référence.

Cette étape de contrôle 13 comprend ensuite une sous-étape de détermination 14 d'au moins une mesure d'un signal résultant de l'acquisition de la caractéristique d'attribut corporel du porteur. Lors de cette sous-étape 14, pendant une durée déterminée configurable, les éléments d'acquisition 8 recueillent au moins une caractéristique d'attribut corporel du porteur qui est transmise par la suite à l'unité de traitement 2 sous la forme d'un signal.

Par la suite, l'étape de contrôle 13 comprend une sous-étape de comparaison 15 entre ladite au moins une mesure et un seuil de référence. Dans ce procédé, les seuils de référence peuvent être créés, dès lors que le porteur a été authentifié et que son identité est certaine et ce, lors d'une étape de génération 11 de seuils de référence relatifs aux caractéristiques d'attribut corporel du porteur authentifié. Lors de cette étape, une fois le porteur de la montre 100 authentifié, l'unité de traitement 2 détermine au moins une mesure d'un signal résultant de l'acquisition de la caractéristique d'attribut corporel du porteur, mesure qui est ensuite archivée dans les éléments de mémoire de l'unité de traitement 2. Les seuils de référence peuvent être donc déterminées automatiquement par l'unité de traitement 2 ou configurés par le porteur durant un processus de réglage visant à guider le porteur dans la définition de ces seuils de référence.

Cette sous-étape de comparaison 15 comprend une phase de suppression 16 de l'autorisation d'accès audites fonctions de la montre 100 si ladite au moins une mesure est sensiblement différente ou différente du seuil de référence. Dans ce cas de figure, l'accès aux fonctions de la montre 100 est alors supprimé car le porteur et propriétaire de la montre 100 peut ne plus être en sa possession. Dès lors dans ce contexte pour utiliser de nouveau les fonctions de la montre 100, il est nécessaire de s'authentifier.

La sous-étape de comparaison 15 comprend aussi une phase de conservation 17 de l'autorisation d'accès audites fonctions de la montre 100 si ladite au moins une mesure est sensiblement égale ou égale au seuil de référence. Dans ce cas, l'unité de contrôle exécute de nouveau, selon la période déterminée, la sous-étape de détermination 14 pour la même caractéristique d'attribut corporel du porteur ou pour une autre caractéristique d'attribut corporel afin de réaliser une autre fois la sous-étape de comparaison 15.

Ainsi, l'invention permet de contrôler en permanence de manière automatique et non-intrusive l'identité du porteur de la montre notamment dès qu'il est authentifié.

L'invention porte aussi sur un programme d'ordinateur comprenant des instructions de code de programme pour l'exécution des étapes 10 à 17 de ce procédé lorsque ledit programme est exécuté par l'unité de traitement 2 de la montre 100.

## Revendications

1. Procédé de sécurisation d'accès à l'utilisation de fonctions d'une montre (100) comportant les étapes suivantes :
- authentification (10) du porteur de la montre (100) en vue d'autoriser un accès audites fonctions ;
- contrôle (13) à période déterminée de l'évolution d'au moins une caractéristique d'attribut corporel du porteur en vue de conserver/supprimer l'autorisation d'accès audites fonctions.

2. Procédé selon la revendication précédente, **caractérisé en ce que** l'étape de contrôle (13) comprend une sous-étape de détermination (14) d'au moins une mesure d'un signal résultant de l'acquisition de la caractéristique d'attribut corporel du porteur.

3. Procédé selon la revendication précédente, **caractérisé en ce que** l'étape de contrôle (13) comprend une sous-étape de comparaison (15) entre ladite au moins une mesure et un seuil de référence.

4. Procédé selon la revendication précédente, **caractérisé en ce que** la sous-étape de comparaison (15) comprend une phase de suppression (16) de l'autorisation d'accès audites fonctions de la montre (100) si ladite au moins une mesure est sensiblement différente ou différente du seuil de référence.

5. Procédé selon la revendication 3, **caractérisé en ce que** la sous-étape de comparaison (15) comprend une phase de conservation (17) de l'autorisation d'accès audites fonctions de la montre (100) si ladite au moins une mesure est sensiblement égale ou égale au seuil de référence.

6. Procédé selon l'une quelconque des revendications précédentes, **caractérisé en ce qu'**il comprend une étape de définition (12) d'au moins une caractéristique d'attribut corporel du porteur dont l'évolution est à contrôler.

7. Procédé selon l'une quelconque des revendications précédentes, **caractérisé en ce qu'**il comprend une étape de génération (11) de seuils de référence relatifs aux caractéristiques d'attribut corporel du porteur authentifié.

8. Procédé selon l'une quelconque des revendications précédentes, **caractérisé en ce que** la caractéristique d'attribut corporel du porteur est choisie parmi les caractéristiques physiologiques, d'expressions corporelles et/ou biométriques du porteur.

9. Système de sécurisation d'accès à l'utilisation de fonctions d'une montre (100) mettant en oeuvre le procédé selon l'une quelconque des revendications précédentes, le système (1) comprenant les éléments suivants reliés entre eux : une unité de traitement (2), au moins un élément d'acquisition de caractéristiques d'attribut corporel du porteur (8), une interface de saisie (9) et des interfaces de diffusion d'une information visuelle et/ou sonore (3, 4).

10. Montre (100) comportant un système (1) selon la revendication précédente.

11. Montre (100) selon la revendication précédente, **caractérisée en ce qu'**elle est une montre (100) mécanique connectée.

12. Programme d'ordinateur comprenant des instructions de code de programme pour l'exécution des étapes (10 à 17) du procédé selon l'une quelconque des revendications 1 à 8 lorsque ledit programme est exécuté par une unité de traitement (2).
